(19) Europäisches Patentamt
European Patent Office
Office européen des brevets

(11) EP 2 566 458 B1

(12) EUROPEAN PATENT SPECIFICATION

(45) Date of publication and mention of the grant of the patent:
**05.08.2015 Bulletin 2015/32**

(21) Application number: **11721665.5**

(22) Date of filing: **02.05.2011**

(51) Int Cl.:
***A61K 9/10*** (2006.01) ***A61K 31/545*** (2006.01)
***A61K 31/546*** (2006.01) ***A61K 47/12*** (2006.01)
***A61K 9/00*** (2006.01)

(86) International application number:
**PCT/TR2011/000131**

(87) International publication number:
**WO 2011/139254 (10.11.2011 Gazette 2011/45)**

# (54) PHARMACEUTICAL FORMULATIONS COMPISING CEFUROXIME AXETIL

PHARMAZEUTISCHE FORMULIERUNGEN MIT CEFUROXIM-AXETIL

FORMULATIONS PHARMACEUTIQUES COMPRENANT DU CÉFUROXIME AXÉTIL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.05.2010 TR 201003543**

(43) Date of publication of application:
**13.03.2013 Bulletin 2013/11**

(73) Proprietor: **Bilgic, Mahmut**
**34220 Esenler-Istanbul (TR)**

(72) Inventor: **Bilgic, Mahmut**
**34220 Esenler-Istanbul (TR)**

(56) References cited:
**WO-A1-00/56286 WO-A1-99/44614**
**GB-A- 1 254 148 US-A1- 2009 175 952**

- **ZELALEM AYENEW ET AL: "Trends in Pharmaceutical Taste Masking Technologies: A Patent Review", RECENT PATENTS ON DRUG DELIVERY & FORMULATION, vol. 3, 1 January 2009 (2009-01-01), pages 26-39, XP55020918,**

Remarks:
The file contains technical information submitted after the application was filed and not included in this specification

EP 2 566 458 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to pharmaceutical compositions comprising (Z)-3-Carboxymethyl-7-(2-(2-furyl)-2-methoxyiminoacetylamino)-3-sefem-4-carboxylic acid which is named as cefuroxime axetil or any pharmaceutically acceptable derivative thereof, and the use of these compositions in the treatment of bacterial infections.

Cefuroxime axetil shown in *formula 1* is a second generation cephalosporin which was first disclosed in the patent numbered US3974153.

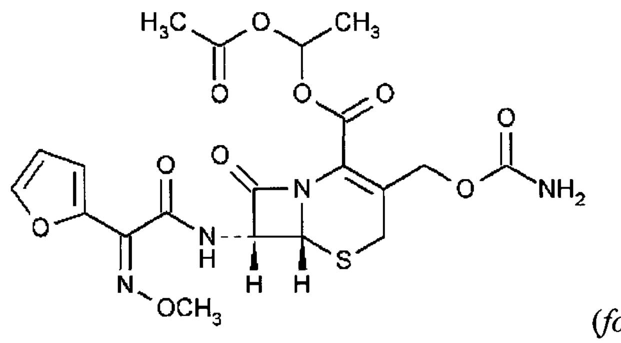

*(formula 1)*

Cefuroxime axetil is a broad spectrum cephalosporin antibiotic against gram negative and gram positive bacteria. Three different polymorphs of it have been described in the prior art. These are the crystal form having a melting point of approximately 180°C, the amorphous form having a melting point of approximately 135°C and the amorphous form having a melting point of approximately 70°C. Cefuroxime axetil in both crystalline form and the amorphous form has a tendency to form a gelatinous mass when contacted with water. This gel formation leads to poor dissolution of cefuxotime axetil and thus low absorption of it.

Cefuroxime axetil has a very bitter taste which is highly durable and which cannot be easily masked by adding sweeteners, taste regulating agents and flavors. In the prior art, coatings have been suggested for both eliminating the extremely bitter taste of cefuroxime axetil and for preventing contact of cefuroxime axetil with water and form a gelatinous mass.

Antibiotics such as cefuroxime axetil for oral administration are generally in the form of granules which can be administered as a suspension since the suspension dosage forms for administration by the oral route are advantageous in respect of their higher dissolution and lower bitterness. Moreover, they do not present the problems encountered in solid dosage forms. For instance, the patent numbered US20030161888, discloses a composition comprising cefuroxime axetil in particulate form formulated as an oral suspension, the particles being provided with integral coatings of lipid or mixture of lipids which are insoluble in water and which disperse or dissolve on contact with gastrointestinal fluid characterized in that said composition comprises a sweetener system and a texture modifier. In this patent application, it is aimed to develop a cefuroxime axetil formulation in suspension form which is easy to swallow and provides to mask the significant bitter taste of cefuroxime axetil and to improve the mouth feel.

US2009/0175952A1 relates to granules for forming a suspension of cefuroxime axetil, where the taste masking is improves. The use of citric acid and trisodium citrate is shown, however at low weight percentage, i.e. about 1 wt%.

WO99/44614A1 and WO00/56286A1 relate to improves solubility of cefuroxine axetil by preventing gel-formation, however, these do not relate to citric acid and trisodium citrate or any other combintaion of two pH agents to achieve this.

However, the coated particles are prepared by atomizing a dispersion of particulate cefuroxime axetil in the molten lipid and cooling the coated particles. This process is a complicated and time consuming process. Moreover, lipid coating may retard the dissolution of cefuroxime axetil and delay its absorption. Furthermore, they are not sufficient to prevent the gelling problem entirely and to mask the bitter taste of cefuroxime axetil.

As is seen, it is required to develop new formulations comprising cefuroxime axetil in suspension form which are prepared by using an easy conventional method, which can overcome the dissolution problem by preventing the gelling while providing a high absorption of it and which eliminates the bitter taste of cefuroxime axetil.

The inventors have surprisingly found that the formulations pertaining to present invention comprising cefuroxime axetil in suspension form, which are produced easily, prevent not only the gelling problem but also the bitter taste of cefuroxime axetil.

## Description of the Invention:

The subject of the present invention relates to the suspension formulations comprising cefuroxime axetil and/or

hydrates, solvates, esters enantiomers, polymorphs, crystal forms, amorphous forms, salt forms or free base form and/or combinations thereof and the procedures for their preparation. Surprisingly, it has been found that pharmaceutical compositions in suspension form comprising cefuroxime axetil wherein at least two pH agents are used and the ratio of the first pH agent having 46-100% (w/w), preferably 50-80% (w/w), more preferably 55-75% (w/w) water solubility at 25°C and the second pH agent having 1-45% (w/w), preferably 5-35%, more preferably 10-25% water solubility at 25°C is in the range of 15:1 and 1:1 by weight, have achieved to overcome the solubility problem of cefuroxime axetil by preventing the gelling and also eliminate the bitter taste of cefuroxime axetil

According to this, the first aspect of the present invention is the pharmaceutical compositions in suspension form comprising cefuroxime axetil in which at least two pH agents are used and the ratio of the first pH agent having 46-100% (w/w), preferably 50-80% (w/w), more preferably 55-75% (w/w) water solubility at 25°C and the second pH agent having 1-45% (w/w), preferably 5-35%, more preferably 10-25% water solubility at 25°C is in the range of 15:1 and 1:1 by weight.

The term "pH agent having 46-100 % (w/w) water solubility at 25°C" means that the amount of pH agent dissolved in 100 g water is in the range of 46-100 g at 25°C.

According to this aspect, the inventors have seen that the selection of the pH agent according to its solubility and the presence of more than one pH agent in the formulation provide both the increase of the solubility of cefuroxime axetil and masking the bitter taste of it. Cefuroxime which is comprised in the suspension formulation pertaining to the present invention can be in the form of its pharmaceutically acceptable hydrates, solvates enantiomers, polymorphs, crystal forms, amorphous forms, and/or combinations thereof.

The first pH agent having 46-100% (w/w) water solubility at 25°C that is used in the suspension formulation pertaining to the present invention is citric acid.

The second pH agent having 1-45% (w/w) water solubility at 25°C that is used in the suspension formulation pertaining to the present invention is trisodium citrate.

The present invention is the pharmaceutical compositions in suspension form comprising cefuroxime axetil in which citric acid and trisodium citrate are used as pH agents and the ratio of citric acid to trisodium citrate is in the range of 15:1 and 1:1 by weight and the total amount of the pH agents that are used in the suspension formulation pertaining to the present invention is in the range of 3-9% by weight.

The suspension formulation pertaining to the present invention can comprise one or several of the excipients including binders, lubricants, glidants, viscosity agents, disintegrants, diluents, preservatives, flavoring agents, sweeteners, coloring agents, surfactants, antifoam agents and stabilizing agents in addition to active agent and at least two pH agents.

The binder that is used in the pharmaceutical formulation pertaining to the present invention can be selected from, but not limited to, a group comprising starches such as potato starch, corn starch, wheat starch; sugars such as sucrose, glucose, dextrose, lactose, maltodextrin; natural and synthetic gums; gelatin; cellulose derivatives such as microcrystalline cellulose, HPC, HEC, HPMC, carboxymethyl cellulose, methyl cellulose, ethyl cellulose; polyvinylpyrrolidone (povidone), polyethylene glycol (PEG); waxes; calcium carbonate; calcium phosphate; alcohols such as sorbitol, xylitol, mannitol, and water or a combination thereof.

The lubricant that is used in the pharmaceutical formulation pertaining to the present invention can be selected from, but not limited to, a group comprising talc, magnesium stearate, stearic acid, sodium stearil fumarate, polyoxyethylene glycol, leucine, alanine, glycine, sodium benzoate, sodium acetate, fumaric acid or a combination thereof.

The disintegrant that is used in the pharmaceutical formulation pertaining to the present invention can be selected from, but not limited to, a group comprising starches such as potato starch, corn starch, wheat starch, pregelatinized starch, sodium starch glycolate; cellulose derivatives such as croscarmellose sodium or microcrystalline cellulose; polyvinylpyrrolidone; crospovidone; alginic acid and its salts; clays such as xanthan gum or Veegum; ion exchange resins or a combination thereof.

The diluent that is used in the pharmaceutical formulation pertaining to the present invention can be selected from, but not limited to, a group comprising lactose, maltose, dextrin, maltodextrin, mannitol, sorbitol, starch and a combination thereof.

The glidant that is be used in the pharmaceutical formulation pertaining to the present invention can be selected from, but not limited to, a group comprising magnesium silicate, silicon dioxide, starch, talc, aerosil 200, tribasic calcium phosphate or a combination thereof.

The flavoring agent that is used in the pharmaceutical formulation pertaining to the present invention can be selected, but not limited to, a group comprising natural aroma oils (peppermint oil, wintergreen oil, clove bud oil, parsley oil, eucalyptus oil, lemon oil, orange oil), menthol, menthane, anethole, methyl salicylate, eucalyptol, cinnamon, 1-methyl acetate, sage, eugenol, oxanone, alpha irisone, marjoram, lemon, orange, blackberry, propenyl guaetol acetyl, cinnamon, vanilla, thymol, linalol, cinnamaldehyde glycerol acetal, N-substituted p-menthane-3-carboxamide, 3,1-methoxy propane 1,2-diol or a combination thereof.

The preservative that is used in the pharmaceutical formulation pertaining to the present invention can be

selected, but not limited to, a group comprising propylene glycol, disodium edentate, benzalkonium chloride, benzoic acid, butyl paraben, cetrimide, chlorobutanol, phenyl mercuric acetate, potassium sorbate, sodium benzoate, sorbic acid, methyl paraben or a combination thereof. Preferably, sodium benzoate is used as preservative.

The sweetener that is used in the pharmaceutical formulation pertaining to the present invention can be selected from, but not limited to, a group comprising sucralose, sucrose, fructose, glucose, galactose, xylose, dextrose, laevulose, lactose, maltose, maltodextrin, mannitol, maltitol, maltol, sorbitol, xylitol, erythritol, lactitol, isomalt, corn syrup, saccharine, saccharine salts, acesulfame potassium, aspartame, D-tryptophane, monoammonium glycyrrhizinate, neohesperidin dihydrochalcone, thaumatin, neotame, alitame, stevioside and cyclamates or a combination thereof.

The viscosity agent that is used in the pharmaceutical formulation pertaining to the present invention can be selected from a group comprising carboxymethyl cellulose, methyl cellulose, xanthan gum, gummi tragacanthae, gum arabic, collidone, agar-agar, bentonite, hydroxyethyl cellulose or a combination thereof.

The inventors have observed that the amount of the viscosity agent used in the formulation is a parameter that has an influence on the solubility of cefuroxime axetil by decreasing the formulation of gelatinous mass. In the case that the viscosity agent is used in an amount of 0.1-4% by weight in the formulation, the increase in the solubility of cefuroxime axetil can be achieved to some extent.

Accordingly, the present invention relates to the pharmaceutical compositions comprising the viscosity agent in an amount of 0.1-4% by weight.

According to the present invention, the formulation in suspension form pertaining to the present invention comprises;

- Cefuroxime axetil in the range of 1-30% by weight
- pH agent in the range of 1-20% by weight
- Viscosity agent in the range of 0.1-4% by weight
- Lubricant in the range of 0-5% by weight
- Sweetener in the range of 20-90% by weight
- Flavoring agent in the range of %0.5-5 by weight
- Glidant in the range of %0.1-3 by weight
- Preservative in the range of %0.05-4 by weight

According to another aspect, the present invention relates to the use of the pharmaceutical compositions in suspension form comprising cefuroxime axetil and at least two pH agents in the treatment of upper respiratory infections such as ear, nose, throat, otitis media, sinusitis, tonsillitis, pharyngitis; lower respiratory tract infections such as pyelonephritis, cystitis and urethritis; skin or soft tissue infections such as froncle, pyoderma, impetigo in the treatment and prophylaxis of gonorrhea and lyme diseases.

The suspension formulation pertaining to the present invention corresponds to powders or granules for the preparation of suspensions and/or dispersed forms of these powders and granules directly with liquid.

According to another aspect, the present invention relates to the process for the preparation of the suspension formulations comprising cefuroxime axetil pertaining to the present invention comprising the steps of mixing cefuroxime axetil , pH agents, sweetener, viscosity agent, preservative, lubricant, glidant and flavoring agent; sieving them and finally filling the mixture into glass bottles.

**Example 1**

| Component name | Amount (% by weight) |
|---|---|
| Cefuroxime axetil | 8% |
| *Citric acid | 3% |
| *Trisodium citrate | 0.3% |
| Preservative | 0.2% |
| Viscosity agent | 1% |
| Sweetener | 84% |
| Glidant | 0.5% |
| Lubricant | 1% |

(continued)

| Component name | Amount (% by weight) |
|---|---|
| Flavoring agent | 2% |
| *Citric acid: trisodium citrate ratio is 10:1. | |

Cefuroxime axetil, citric acid, trisodium citrate, sweetener, viscosity agent, preservative, lubricant, glidant and flavoring agent are mixed; then the mixture is sieved and finally the mixture is filled into glass bottles.

**Comparative Example 1**

| Component name | Amount (% by weight) |
|---|---|
| Cefuroxime axetil | 6.5% |
| *Citric acid | 6% |
| *Trisodium citrate | 0.3% |
| Preservative | 0.2% |
| Viscosity agent | 0.5% |
| Sweetener | 84% |
| Glidant | 1% |
| Lubricant | 0.5% |
| Flavoring agent | 1.0% |
| *Citric acid: trisodium citrate ratio is 20:1. | |

Cefuroxime axetil, citric acid, trisodium citrate, sweetener, viscosity agent, preservative, lubricant, glidant and flavoring agent are mixed; then the mixture is sieved and finally the mixture is filled into glass bottles.

**Comparative Example 2**

| Component name | Amount (% by weight) |
|---|---|
| Cefuroxime axetil | 7.5% |
| Citric acid | 5% |
| Preservative | 0.2% |
| Viscosity agent | 1% |
| Sweetener | 84% |
| Glidant | 1% |
| Lubricant | 0.3% |
| Flavoring agent | 1.0% |

Cefuroxime axetil, citric acid, sweetener, viscosity agent, preservative, lubricant, glidant and flavoring agent are mixed; then the mixture is sieved and finally the mixture is filled into glass bottles.

**Comparative Example 3**

| Component name | Amount (% by weight) |
|---|---|
| Cefuroxime axetil | 7% |
| Trisodium citrate | 4.5% |
| Preservative | 0.7% |
| Viscosity agent | 0.5% |
| Sweetener | 84% |
| Glidant | 1% |
| Lubricant | 0.8% |
| Flavoring agent | 1.5% |

Cefuroxime axetil, trisodium citrate, sweetener, viscosity agent, preservative, lubricant, glidant and flavoring agent are mixed; then the mixture is sieved and finally the mixture is filled into glass bottles.

**Results :**

The inventors have studied on the dissolution data and bitterness values of the compositions illustrated in each example given above. According to the studies based on the comparison of their dissolution data and the bitterness values, the results represented in Tables 1-6 are observed. Table 1 indicates the comparative data of dissolution of the compositions for each example. In Table 2 to Table 5, the results of the bitterness values of each cefuroxime axetil compositions which are calculated according to the European Pharmacopoeia 5.0 (2.8.15, pg. 221) are shown.

**Table 1:** Comparative Data of Dissolution of Cefuroxime Axetil Compositions in Suspension Form

| Sample | Time (min) | % dissolved amount of composition |
|---|---|---|
| Composition of Example 1 | 45 | 95% |
| Composition of Comparative Example 1 | 45 | 75% |
| Composition of Comparative Example 2 | 45 | 65% |
| Composition of Comparative Example 3 | 45 | 60% |

The results of the dissolution data of compositions of Example 1, Comparative example 1, Comparative example 2, Comparative example 3 clearly indicate that the suspension of the present invention in which citric acid and trisodium citrate are used and citric acid: trisodium ratio by weight is 10:1, as shown in example 1, has a higher dissolution rate compared to the others.

**Table 2:** Results of the Taste Trial for Cefuroxime Axetil Composition of Example 1

| Panelist* | k* | Y* | X* | Bitterness Value* |
|---|---|---|---|---|
| 1 | 0.76 | 10000 | 6 | 12667 |
| 2 | 0.84 | 100 | 8 | 105 |
| 3 | 0.72 | 10000 | 8 | 9000 |
| 4 | 0.8 | 50000 | 3 | 133335 |
| 5 | 0.76 | 100 | 6 | 127 |
| 6 | 0.72 | 10000 | 8 | 9000 |

(continued)

| Panelist* | k* | Y* | X* | Bitterness Value* |
|---|---|---|---|---|
| Average | | | | **27372** |
| Panelist*: a member of a taste panel of six people<br>k*: the correction factor for each panelist<br>Y*: Dilution Factor (DF) of solution D which is a diluted solution having still a bitter test<br>X*: number of mililitres of solution D which, when diluted to 10 ml with water, still has a bitter taste Bitterness value: a value computed by using the expression of $\frac{Y\times k}{X\times 0.1}$ | | | | |

**Table 3:** Results of the Taste Trial for Cefuroxime Axetil Composition of Comparative Example 1

| Panelist* | k* | Y* | X* | Bitterness Value* |
|---|---|---|---|---|
| 1 | 0.76 | 10000 | 6 | 12667 |
| 2 | 0.84 | 50000 | 3 | 140000 |
| 3 | 0.72 | 100 | 3 | 240 |
| 4 | 0.8 | 100 | 2 | 400 |
| 5 | 0.76 | 10000 | 3 | 25334 |
| 6 | 0.72 | 50000 | 3 | 120000 |
| Average | | | | **49773** |

**Table 4:** Results of the Taste Trial for Cefuroxime Axetil Composition of Comparative Example 2

| Panelist* | k* | Y* | X* | Bitterness Value* |
|---|---|---|---|---|
| 1 | 0.76 | 10000 | 2 | 38000 |
| 2 | 0.84 | 10000 | 1.5 | 56000 |
| 3 | 0.72 | 100000 | 3 | 240000 |
| 4 | 0.8 | 10000 | 1.5 | 53334 |
| 5 | 0.76 | 10000 | 1.5 | 50667 |
| 6 | 0.72 | 10000 | 2 | 36000 |
| Average | | | | **79000** |

**Table 5:** Results of the Taste Trial for Cefuroxime Axetil Composition of Comparative Example 3

| Panelist* | k* | Y* | X* | Bitterness Value* |
|---|---|---|---|---|
| 1 | 0.76 | 10000 | 2 | 38000 |
| 2 | 0.84 | 100000 | 3 | 280000 |
| 3 | 0.72 | 10000 | 1.5 | 48000 |
| 4 | 0.8 | 10000 | 1.2 | 66667 |
| 5 | 0.76 | 10000 | 1.5 | 50667 |
| 6 | 0.72 | 10000 | 2 | 36000 |
| Average | | | | **86556** |

Taste trials were performed by a taste panel comprising 6 persons. The average bitterness value of each suspension of the compositions of Example 1, Comparative example 1, Comparative example 2, Comparative example 3 are calculated according to the European Pharmacopoeia 5.0. Six panelists assessed these compositions which are represented in Tables 2-5. The results clearly show that average bitterness value of the composition of example 1 is the lowest one. Therefore, it can be concluded that suspension of the present invention in which citric acid and trisodium citrate are used and citric acid: trisodium ratio by weight is 10:1 shown in example 1 is much preferred for its low bitterness value.

## Claims

1. A formulation in suspension form comprising cefuroxime axetil **characterized in that**,

    - citric acid is used as the first pH agent and trisodium citrate is used as the second pH agent,
    - the total amount of the pH agents is in the range of 3-9 % by weight and
    - the ratio of the first pH agent having 46-100% (w/w) water solubility at 25°C and the second pH agent having 1-45% (w/w) water solubility at 25°C is in the range of 15:1 and 1:1 by weight.

2. The formulation according to claim 1, wherein cefuroxime axetil is in the form of its pharmaceutically acceptable hydrates, solvates, enantiomers, polymorphs, crystal forms, amorphous forms, and/or a combination thereof.

3. The formulation according to claims 1 - 2, wherein said formulation comprises one or several of the excipients including binders, lubricants, glidants, viscosity agents, disintegrants, diluents, preservatives, flavoring agents, sweeteners, coloring agents, surfactants, antifoam agents and stabilizing agents.

4. The formulation according to claim 3, wherein viscosity agent is chosen from a group comprising carboxymethyl cellulose, methyl cellulose, xanthan gum, gummi tragacanthae, gum arabic, collidone, agar-agar, bentonite, hydroxyethyl cellulose or combinations thereof.

5. The formulation according to claim 4, wherein the amount of the viscosity agent that said formulation contains is in the range of 0.1-4% by weight.

6. The formulation according to claims 1-5, wherein said formulation comprises;

    - Cefuroxime axetil in the range of 1-30% by weight
    - pH agent in the range of 3-9 % by weight
    - Viscosity agent in the range of 0.1-4% by weight
    - Lubricant in the range of 0-5% by weight
    - Sweetener in the range of 20-90% by weight
    - Flavoring agent in the range of %0.5-5 by weight
    - Glidant in the range of %0.1-3 by weight
    - Preservative in the range of %0.05-4 by weight

7. The process for the preparation of suspension formulations comprising cefuroxime axetil pertaining to the present invention according to claims 1-6 wherein said process comprises the steps of mixing cefuroxime axetil, pH agents, sweetener, viscosity agent, preservative, lubricant, glidant and flavoring agent; sieving them and finally filling the mixture into glass bottles.

## Patentansprüche

1. Eine Formulierung in Form einer Suspension, die Cefuroximaxetil beinhaltet und **dadurch gekennzeichnet, dass**

    - Zitronensäure als erstes pH Mittel und Trinatriumcitrat als zweites pH Mittel verwendet wird;
    - die Gesamtmenge der pH Mittel im Bereich von 3-9% nach Gewicht liegt und
    - das Verhältnis des ersten pH Mittels mit der Wasserlöslichkeit von 46-100 % (w / w) bei 25°C und das zweite pH Mittel mit der Wasserlöslichkeit von 1-45% (w / w) bei 25°C im Bereich von 15:1 und 1:1 nach Gewicht liegen.

**2.** Die Formulierung nach Anspruch 1, wobei Cefuroximaxetil in der Form seiner pharmazeutisch annehmbaren Hydrate, Solvate, Enantiomere, Polymorphen, Kristallformen, amorphen Formen, und / oder eine Kombination davon vorhanden ist.

**3.** Die Formulierung nach den Ansprüchen 1-2, wobei die genannte Formulierung eines oder einige der Hilfsstoffe einschließlich Bindemittel, Schmiermittel, Gleitmittel, Viskositätsmittel, Sprengmittel, Verdünnungsmittel, Konservierungsmittel, Geschmacksstoffe, Süßungsmittel, Färbemittel, Tenside, Antischaummittel und Stabilisierungsmittel beinhaltet.

**4.** Die Formulierung nach Anspruch 3, wobei Viskositätsmittel aus einer Gruppe bestehend aus Carboxymethylzellulose, Methylcellulose , Xanthangummi, Gummi tragacanthae, Gummi arabicum, Kollidon, Agar-Agar, Bentonit, Hydroxyethylzellulose oder Kombinationen davon ausgewählt wird.

**5.** Die Formulierung nach Anspruch 4, wobei die Menge des Viskositätsmittels, die die genannte Formulierung beinhaltet, im Bereich von 0,1-4 nach Gewicht liegt.

**6.** Die Formulierung nach Ansprüche 1-5, wobei die genannteFormulierung

- Cefuroximaxetil im Bereich von 1-30% nach Gewicht;
- pH Mittel im Bereich von 3-9% nach Gewicht;
- Viskositätsmittel im Bereich von 0,1-4% nach Gewicht;
- Schmiermittel im Bereich von 0-5% nach Gewicht;
- Süßstoff im Bereich von 20-90% nach Gewicht;
- Geschmacksmittel im Bereich von 0,5-5% nach Gewicht;
- Gleitmittel im Bereich von 0,1-3% nach Gewicht und
- Konservierungsmittel im Bereich von 0,05-4% nach Gewicht beinhaltet.

**7.** Das Verfahren zur Herstellung von Suspensionsformulierungen enthaltend Cefuroximaxetil und betreffend der vorliegenden Erfindung gemäß den Ansprüchen 1 - 6, wobei das genannteVerfahren die Schritte des Mischens und des Siebens von Cefuroximaxetil, pH Mitteln, Süßstoffen, der Viskosität, Konservierungsmitteln, Gleitmitteln, Fließregulierungs- und Geschmacksmitteln und schließlich Füllung der Mischung in Glasflaschenumfasst.

**Revendications**

**1.** Une formulation sous la forme d'un suspension comprenant le céfuroxime axétil **caractérisé en ce que** l'acide citrique est utilisé comme le premier agent de pH et le citrate trisodique est utilisé comme le second agent de pH, la quantité totale des agents de pH est dans la plage de 3-9% en poids, et le rapport du premier agent de pH ayant 46 à 100% (p/p) de solubilité dans l'eau à 25°C et le second agent de pH comportant 1-45% (p/p) solubilité dans l'eau à 25°C est dans la plage de 15:1 et 1:1 en poids.

**2.** La formulation selon la revendication 1, dans laquelle le céfuroxime axétil se présente sous la forme de ses hydrates, ses solvates, ses énantiomères, ses polymorphes, ses formes cristallines, ses formes amorphes pharmaceutiquement acceptables et/ou d'une combinaison de ceux-ci.

**3.** La formulation selon les revendications 1-2, dans lesquelles ladite formulation comprend un ou plusieurs des excipients, y compris des liants, des lubrifiants, des agents de glissement, des agents de viscosité, des désintégrants, des diluants, des conservateurs, des agents aromatisants, des édulcorants, des agents colorants, des tensioactifs, des agents antimousse et des agents de stabilisation.

**4.** La formulation selon la revendication 3, dans laquelle l'agent de viscosité est choisi d'un groupe comprenant la carboxyméthylcellulose, la méthylcellulose, la gomme xanthane, la gomme tragacanthae, la gomme arabique, la collidone, de l'agar-agar, la bentonite, la cellulose d'hydroxyéthyle ou les combinaisons de ceux-ci.

**5.** La formulation selon la revendication 4, dans laquelle la quantité de l'agent de viscosité que ladite formulation contient est dans la plage de 0.1-4% en poids.

**6.** La formulation selon les revendications 1-5, dans lesquelles ladite formulation comprend;

le céfuroxime axetil dans la plage de 1-30% en poids, l'agent de pH dans la plage de 3-9 % en poids, l'agent de viscosité dans la plage de 0.1-4% en poids, le lubrifiant dans la plage de 0-5% en poids, l'édulcorant dans la plage de 20-90% en poids, l'agent aromatisant dans la plage de 0.5-5% en poids, le glissant dans la plage de 0.1-3% en poids, le conservateur dans la plage de 0.05-4% en poids.

**7.** Le procédé de préparation des formulations de suspensions comprenant du cefuroxime axétil se rapportant à la présente invention selon les revendications 1-6, dans lesquelles ledit procédé comprend les étapes de mélanger de céfuroxime axétil, les agents de pH, l'édulcorant, l'agent de viscosité, le conservateur, le lubrifiant, le glissant et l'agent aromatisant; de les tamiser et finalement de remplir du mélange dans des bouteilles en verre.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3974153 A
- US 20030161888 A
- US 20090175952 A1
- WO 9944614 A1
- WO 0056286 A1